# EUROPÄISCHE PATENTANMELDUNG

(11) **EP 0 614 035 A1**
(43) Veröffentlichungstag der Anmeldung: **07.09.1994**
(21) Anmeldenummer: 94103134.6
(22) Anmeldetag: 02.03.1994
(51) Int. Cl.: F16M 11/06

(54) **Deckenstativ**

(30) Priorität: 04.03.1993 DE 4306803
(71) Anmelder: Kreuzer GmbH + Co. OHG, D-82178 Puchheim (DE)
(72) Erfinder: Martin, Karl-Heinz, D-82284 Grafrath (DE)
(74) Vertreter: Prüfer, Lutz H., Dipl.-Phys.

(57) **Zusammenfassung**

Es soll ein Deckenstativ (1) zum Tragen von medizinischen Geräten (6) und Zuführen von Versorgungseinrichtungen im Medizinbereich mit Stativarmen (3, 4) und mit einer Tragsäule (5) so ausgebildet sein, daß ihre Herstellung einfach und kostengünstig ist. Zum diesem Zweck sind die Stativarme (3, 4) aus einem Strangpreßprofil mit einem im wesentlichen rechteckigen Querschnitt mit jeweils einem sich parallel zu den Seitenwandungen (10, 11) erstreckenden und von diesen einen Abstand aufweisenden Längsstegen (14, 15) zwischen Ober- und Unterwand (8, 9) und einem zwischen Ober- und Unterwand und den Stegen gebildeten Kanal (18) für die Versorgungsleitungen ausgebildet.

## Beschreibung

Die Erfindung betrifft ein Deckenstativ nach dem Oberbegriff des Patentanspruches 1.

Ein solches Deckenstativ wird insbesondere im medizinischen Bereich zum Tragen von Geräten im Operationsraum oder ähnlichem verwendet.

Bei derartigen bekannten Deckenstativen ist der Stativarm als Gußteil ausgebildet. Die Herstellung solcher Deckenstativarme ist aufwendig.

Aufgabe der Erfindung ist es, ein einfach ausgebildetes und kostengünstig herzustellendes Deckenstativ zu schaffen.

Diese Aufgabe wird durch das in Patentanspruch 1 gekennzeichnete Deckenstativ gelöst.

Weiterbildungen der Erfindung sind in den Unteransprüchen gekennzeichnet.

Weitere Merkmale und Zweckmäßigkeiten der Erfindung ergeben sich aus der Beschreibung eines Ausführungsbeispieles anhand der Figuren.

Von den Figuren zeigen:
- Fig. 1: eine schematische Seitenansicht eines Deckenstatives;
- Fig. 2: einen Schnitt entlang der Linie II-II in Fig. 1;
- Fig. 3: eine Seitenansicht eines Stativarmes mit abgenommener Endkappe;
- Fig. 4: eine Draufsicht auf den in Fig. 3 gezeigten Stativarm mit abgenommener Endkappe; und
- Fig. 5: einen Schnitt entlang der Linie V-V in Fig. 3.

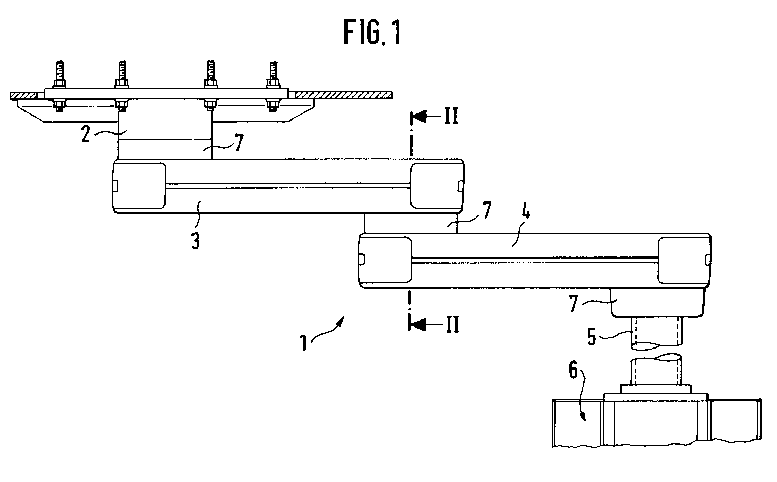

Das in Fig. 1 gezeigte Deckenstativ weist in bekannter Weise eine Deckenhalterung 2, einen damit verbundenen ersten Stativarm 3, einen mit diesem verbundenen zweiten Stativarm 4 und eine von letzterem getragene Säule 5 zum Aufnehmen von Lasten in Form medizinischer Instrumente 6 auf. Die Deckenhalterung 2 ist mit dem ersten Stativarm 3 und dieser wiederum mit dem zweiten Stativarm 4 und mit der Säule 5 jeweils über ein Drehgelenk 7 verbunden.

Jeder der beiden Stativarme 3, 4 ist als Strangpreßprofil mit im wesentlichen rechteckigem Querschnitt ausgebildet. Dabei ist der Querschnitt so gewählt, daß die Oberwand 8 und die Unterwand 9 von den Breitseiten und die beiden Seitenwandungen 10, 11 von den Schmalseiten gebildet werden. Die Arme sind zu ihrer Mittenachse 12 symmetrisch ausgebildet, so daß im weiteren nur eine Hälfte beschrieben wird. Im wesentlichen in der Mitte der Seitenwandungen ist jeweils eine T-förmige Nut 13 vorgesehen, die sich parallel zur Längsachse des Stativarmes erstreckt. Ferner sind im Inneren der Profile in einem kleinen Abstand von den jeweiligen Seitenwandungen 10, 11 Längsstege 14, 15 vorgesehen, die sich im wesentlichen senkrecht zu der Oberwand bzw. Unterwand und im wesenlichen parallel zu den Seitenwandungen als gerader Steg erstrecken. Die Längsstege sind jeweils über sich im wesentlichen parallel zur Oberwand 8 bzw. Unterwand 9 erstreckende Längsstege 16, 17 mit der Rückseite der T-Nut 13 verbunden. Auf diese Weise erreicht der mit Hilfe der Strangpreßtechnik hergestellte Stativarm 3, 4 eine hohe Stabilität und stellt im Inneren zwischen Oberwand 8 und Unterwand 9 und den Längsstegen 14, 15 einen Kanal 18 für die Hindurchführung von Versorgungsleitungen aller Art bereit.

Die T-Nuten 13 dienen gleichzeitig zur Aufnahme von Halterungen, die in der Art von nicht gezeigten Kopfschrauben mit ihrem Kopf in den erweiterten Bereich der jeweiligen Nut 13 eingesetzt und in dieser verschiebbar arretiert sind.

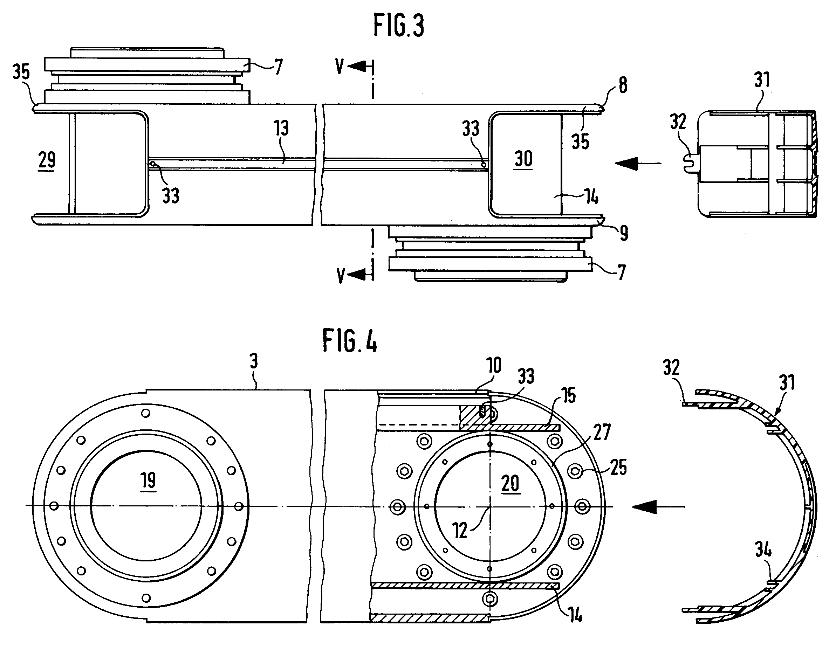

Wie am besten aus Fig. 4 ersichtlich ist, weist der Stativarm 3 an seinem mit der Halterung 2 verbundenen Ende oben eine kreisförmig ausgebildete Ausnehmung 19 und auf seinem gegenüberliegenden, mit dem zweiten Stativarm 4 verbundenen Ende unten eine kreisförmige Ausnehmung 20 auf. Der Durchmesser der Ausnehmungen ist so gewählt, daß sich die Ausnehmungen über die gesamte Breite zwischen den beiden einander zugewandten Längsstegen 14, 15 erstreckt, um so die größtmögliche Kanaldurchführung aus dem Kanal 18 zu dem zweiten Stativarm 4 zu haben. Der zweite Stativarm hat den gleichen Aufbau, um eine entsprechende Kanaldurchführung zu der Säule 5 zu gewährleisten.

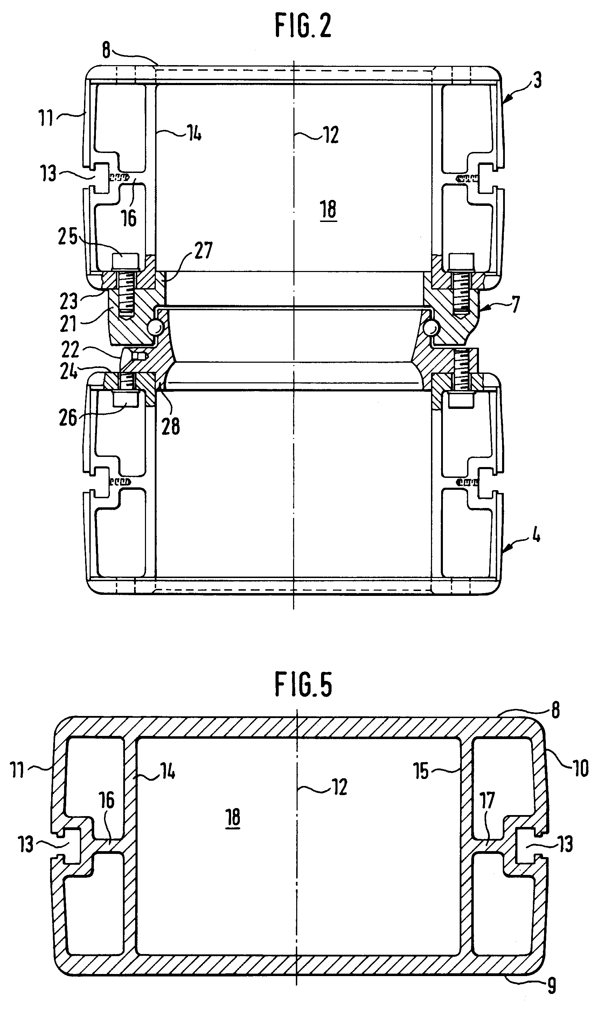

Jedes Drehgelenk 7 weist eine mit dem obenliegenden Teil verbundene Lagerhälfte 21 und eine damit zusammenwirkende, mit dem untenliegenden Teil verbundene zweite Lagerhälfte 22 auf. Die Verbindung der Lagerhälften 21, 22 mit den zugehörigen Teilen erfolgt jeweils durch geeignete Verschraubung. Wie am besten aus Fig. 2 ersichtlich ist, sind die der jeweiligen damit zu verbindenden Lagerhälfte zugewandten Ränder 23, 24 der Ausnehmungen 19, 20 im wesentlichen plan bearbeitet, so daß sich ihre Ebene senkrecht zur Mittenachse 12 erstreckt. Die damit zu verbindenden Lagerteile 21, 22 weisen den Rändern 23, 24 jeweils entsprechende ringförmige Widerlagerflächen auf, mit denen sie an den jeweiligen Rändern 23, 24 anliegen und mittels Schrauben 25, 26 fest verbunden sind. Zusätzlich weist jedes Lagerteil einen kragenartigen zylindrischen Abschnitt 27, 28 auf, der jeweils in die von dem ringförmigen Rand 23, 24 begrenzte Ausnehmung 19 bzw. 20 hineinragt. Jeder kragenartige zylindrische Abschnitt 27, 28 weist eine äußere Zylinderfläche auf, deren Durchmesser so in Abhängigkeit von dem Durchmesser der Ausnehmungen 19, 20 und damit dem Abstand der Längsstege 14, 15 bemessen ist, daß das jeweilige Lagerteil mit seinem kragenartigen zylindrischen Abschnitt im Paßsitz mit dem jeweiligen Rand verbunden ist. Durch das Vorsehen dieser kragenartigen zylindrischen Abschnitte werden von dem jeweiligen Stativarm auf das Lager ausgeübte Kippkräfte aufgenommen.

Wie am besten aus den Figuren 3 und 4 ersichtlich ist, sind die beiden Enden eines jeden Stativarmes halbkreisförmig ausgebildet mit einem Radius, der gleich der halben Breite des Profiles ist und dessen Mittelpunkt durch die Mittenachse 12 geht. Dadurch entstehen an den beiden Enden Öffnungen 29, 30, über die die Lagerhälften und die diese mit den Stativarmen verbindenden Schrauben sowie die durch den jeweiligen Kanal und die Öffnungen hindurchzuführenden Versorgungsleitungen frei zugänglich sind. Zum Abdecken der Öffnungen sind auf jeder Seite halbzylinderförmige Kappen 31 vorgesehen, von denen nur eine gezeigt ist. Der Radius des Halbzylinders entspricht dem Radius der jeweiligen abzudeckenden Enden. Die Kappen 31 weisen auf beiden Seiten über den Rand hervorstehende Laschen 32 mit je einem Schlitz auf, welche in die T-Nut 13 eingeschoben werden und dort in der Gewindebohrung 33 mit einer Schraube befestigt werden. Die Laschen 32 gewährleisten zusammen mit Laschen 34, welche beim Einschieben der Kappe 31 sich über die Längsstege 15 schieben, einen stabilen Sitz der Kappe um den um die gesamte Öffnung 30, 31 verlaufenden Ansatz 35 und verhindern über die Armoberfläche herausragende Kappenteile, so daß überall glatte Flächen gebildet sind. Die Laschen 32, 34 tragen gleichzeitig zur Stabilisierung der Kappen 31 bei.

Auf diese Weise entsteht ein Stativ mit hoher Festigkeit, welches durch die abnehmbaren End-Kappen 31 eine einfache Handhabung für Inspektion und Arbeiten in seinem Inneren zuläßt. Weiterhin sind die Forderungen der Raumlufttechnik nach glatten Flächen erfüllt.

## Patentansprüche

1. Deckenstativ (1) mit wenigstens einem ersten Stativarm (3, 4), einer Säule (5) zum Tragen von Instrumenten (6) und ähnlichem und im Inneren angeordneten Versorgungsleitungen für die Instrumente (6) und ähnliches, wobei der eine Stativarm (3) schwenkbar mit einer Deckenhalterung verbunden ist,
gekennzeichnet durch
die Ausbildung des Stativarmes (3, 4) aus einem Strangpreßprofil mit einem im wesentlichen rechteckigen Querschnitt mit jeweils einem sich im wesentlichen parallel zu den Seitenwandungen (10, 11) erstreckenden und von diesen einen Abstand aufweisenden Längsstegen (14, 15) zwischen Ober- und Unterwand (8, 9) und einem zwischen Ober- und Unterwand (8, 9) und den Stegen (14, 15) gebildeten Kanal (18) für die Versorgungsleitungen.

2. Deckenstativ nach Anspruch 1, dadurch gekennzeichnet, daß
wenigstens eine der Seitenwände (10, 11) eine sich in Längsrichtung des Stativarmes erstreckende T-förmige Nut (13) aufweist.

3. Deckenstativ nach Anspruch 2, dadurch gekennzeichnet, daß
das Profil einen den Grund der T-förmigen Nut (13) mit dem benachbarten Längssteg (14, 15) verbindenden Längssteg (16), der sich im wesentlichen senkrecht zu dem benachbarten Längssteg (14, 15) erstreckt, aufweist.

4. Deckenstativ nach einem der Ansprüche 1 bis 3, dadurch gekennzeichnet, daß
ein mit dem ersten Stativarm (3) über ein Drehgelenk (7) verbundener zweiter Stativarm (4) vorgesehen ist, jeder Stativarm (3, 4) an der Stelle des Drehgelenkes (7) eine Ausnehmung (19, 20) aufweist und das Drehgelenk (7) zwei ringförmige Lagerhälften (21, 22) aufweist, die jeweils eine mit dem zugehörigen Stativarm (3, 4) zu verbindende Fläche und einen sich im wesentlichen parallel zur Mittenachse (12) des Lagers erstreckenden Kragen (27, 28) zum Anliegen an der Wandung (14, 15) der jeweiligen Ausnehmung (19, 20) zum Aufnehmen von Kippkräften aufweist.

5. Deckenstativ nach einem der Ansprüche 1 bis 4, dadurch gekennzeichnet, daß
der Stativarm (3, 4) an seinem längsseitigen Ende eine Öffnung (29, 30) aufweist, die durch eine abnehmbare Kappe (31) verschlossen ist.

6. Deckenstativ nach Anspruch 5, dadurch gekennzeichnet, daß
die Kappe (31) mit den T-förmigen Nuten (13) bzw. den Längsstegen (14, 15) in lösbarem Eingriff befindliche Laschen (32, 34) aufweist.
